# EUROPEAN PATENT SPECIFICATION

(11) **EP 0 549 730 B1**
(45) Date of publication and mention of the grant of the patent: **26.07.1995**
(21) Application number: 91920317.4
(22) Date of filing: 12.09.1991
(51) Int. Cl.: A61M 39/02, A61M 39/00

(54) **NEEDLELESS CONNECTOR SAMPLE SITE**
NADELLOSE INJEKTIONSSTELLE
POINT DE PRISE D'ECHANTILLON AVEC RACCORD SANS AIGUILLE

(30) Priority: 18.09.1990 US 584286
(43) Date of publication of application: 07.07.1993
(60) Divisional application: 94203665.8
(73) Proprietor: MEDEX, INC., Hilliard Ohio 43026 (US)
(72) Inventor: FRANK, Thomas, P., Dublin, OH 43017 (US); PATZER, Charles, R., Columbus, OH 43200 (US)
(74) Representative: Findlay, Alice Rosemary (GB)
(86) International application number: US9106604
(87) International publication number: WO9204936

(56) References cited:
- EP-A- 0 309 771
- EP-A- 0 343 953
- WO-A-89/06553
- DE-C- 3 303 718
- US-A- 5 006 114

## Description

### Field of the Invention

This invention relates to sample sites through which fluids are injected or withdrawn from a patient's circulatory system and, more particularly, to such a sample site which does not require use of a needle for injecting or withdrawing samples and in which the access port is easily reached for disinfecting.

### Background of the Invention

Sample sites for obtaining fluid access to a patient's circulatory system are well known. For example, a catheter inserted into a patient's blood vessel may be connected to a sample site such as shown in U.S. A. 4,874,377, the disclosure of which is fully incorporated herein by reference. Alternatively, the sample site may be in-line with tubing connecting the catheter to a blood pressure monitoring transducer and/or a supply of saline or other solution, such as shown in US-A-5148 811, entitled "Method and Apparatus for Sampling Blood," which is a continuation-in-part of US-A-5048537.

Typical of sample sites is that they include a housing having a liquid path extending therein and which is to be coupled to the patient's circulatory system to make the fluid connection such as through a catheter and/or the tubing of an intravenous system, for example, like the blood pressure monitoring apparatus shown in the aforementioned patent applications. A sample site further typically includes an access port through which fluids may be introduced into the patient's circulatory system or from which blood may be withdrawn from the patient, both via the liquid path. By way of example, a needle may be received through a resilient stopper in the access port and on into the liquid path to couple a syringe or the like attached to the needle with the patient's circulatory system for injection/withdrawal of fluids. However, sampling sites requiring use of a needle present significant risks from needle sticks. As will be readily appreciated, needle sticks provide a mechanism for transfer of dangerous diseases, such as hepatitis or AIDS.

To reduce risk of needle sticks, sampling sites have been developed which eliminate needles. Such sampling sites provide an access port adapted for needleless connection with an external fluidic system such as a syringe or tubing. To this end, a cylindrical reservoir is provided on the sample site to provide a confined conduit between the needleless connector such as a male luer connector and a valve assembly within the sample site. One example is the stopcock side port shown in Figure 5 in the aforementioned US-A-5148811 in which the external fluidic system is connected thereto by securing the cuff of a male luer lock connector to the flanged top of a reservoir defined by the cylindrical wall of a female luer connector. Other needleless access ports are provided in which a blunt cannula, such as the tip or taper of a male luer connector, is receivable into the cylindrical reservoir. As shown, for example, in aforementioned U.S.-A-4,874,377, the male luer connector tip impacts the valve assembly at the bottom of the female luer connector reservoir causing the valve to deflect and open under pressure of the tip of the male luer connector thereagainst. As the valve assembly opens, a channel for fluid communication between the liquid path within the sample site and the external fluidic system is created.

While such needleless sample sites reduce or eliminate needle stick problems, the cylindrical reservoir by which to secure the male liner connector to the sample site has introduced its own significant problems. In particular, the reservoir is difficult to clean and has prompted concern about contamination and the dangers of bacterial growth therein.

EP-A-0309771 describes a needleless sample site comprising a housing having a cylindrical aperture extending from an exterior wall through which a blunt cannula enters to make a connection to a liquid path. A seal is located in the aperture which is operable under pressure of the blunt cannula. A gap is provided between the aperture wall and the seal to accommodate expansion of the latter on opening thereof. When the site is not in use, a cover is securable over the housing exterior wall.

A sample site, in accordance with the invention, comprises a housing, a liquid path extending into the housing, an apertured exterior wall on the housing through which a blunt cannula enters the housing to make fluid connection to the liquid path, and, seal means adjacent the exterior wall aperture and presenting an outer surface generally flush the housing exterior wall for normally sealing the aperture against fluid communication with the liquid path, the seal means being openable under pressure of the blunt cannula passing into the aperture whereby to permit fluid connection thereof with the liquid path, the seal means resealing after removal of the blunt cannula, characterised in that a pair of arms extend away from the housing about the aperture for receiving the blunt cannula therethrough and in that the arms are spaced apart to facilitate cleaning of the seal means by providing wiping access between the arms and across the seal means outer surface and adjacent portions of the housing exterior wall.

The present invention provides the benefits of a needleless sample site, while reducing the risk of contamination and bacterial growth. To this end, the reservoir above the valve assembly is eliminated and replaced with arms extending above the access port to provide the function of guiding a blunt cannula such as a male luer connector tip against the seal, but without a reservoir wall to obstruct ready access to the surface of the valve assembly for disinfecting. The arms may be flanged to provide the function of locking a male luer lock connector thereto.

More specifically, the access port is defined by an apertured exterior wall of the sample site housing through which a blunt cannula such as the tip of a male luer connector is to be received. The outer surface of a seal member contained within the housing normally blocks the aperture. The exterior wall adjacent the aperture and the outer surface of the seal member are easily wiped clean and, thus, may be considered to be generally flush in the closed position of the seal member. Consequently, there is no reservoir defined about the aperture and seal surface from which contaminants or bacteria may be difficult to remove. The seal member is deflectable under pressure of the tip of a male luer connector passing into the aperture so as to open the seal member and provide fluid access to the liquid path in the sample site. When the male luer connector is withdrawn, the seal shuts to again block the aperture, but because there is no confined reservoir above the aperture, the exposed surface of the seal member and portions of the exterior wall adjacent the aperture (as well as the flanged arms) are readily accessible to a clinician for purposes of disinfecting the access port, such as by a wiping action between the flanged arms in a direction generally parallel the seal member and/or exterior wall surfaces. Consequently, there is less opportunity for contamination or bacterial growth with the sampling site of the present invention.

A female luer connector function may be provided atop the access port but without the cylindrical reservoir normally provided thereby. To this end, the pair of arms are provided adjacent the access port aperture such as to matingly receive a male luer connector tip therethrough while still providing generally unobstructed wiping access to the outer surface of the seal member and portions of the housing exterior wall adjacent the aperture. The arms may be flanged to lock the male luer connector thereto such that the arms may be seen as separate portions of a female luer connector and may thus be referred to as a split luer.

In one embodiment, the female luer connector portions or flanged arms are fixedly mounted to the housing exterior wall adjacent the aperture such that the inner or confronting surfaces of the flanged arms define an imaginary female luer connector cylinder about the aperture, but without the impediment of a complete cylindrical wall. In a further embodiment, the flanged arms are moveable from an operative position about the aperture to provide the female luer connector function to an inoperative position away from the sample site housing to provide completely unobstructed access to the outer surface of the seal member, as well as to the annular portion of the housing exterior wall completely surrounding the aperture. In accordance with this further embodiment, the flanged arms are mounted to the end of hinge arms so as to be moveable toward and away from the aperture. The hinge arms may be fixedly connected at their extreme ends to the housing and remote from the aperture and normally biased to urge the flanged arms away from the aperture. The hinge arms may then be compressed toward the sample site housing bringing the flanged arms adjacent the aperture for connecting to the male luer connector. Alternatively, the hinge arms may be slidably connected to the housing for sliding the flanged arms between the operative and inoperative positions. With the hinge arms connected to a frame member slidably disposed on a side of the housing opposite the access port, the hinge arms will ride along the side of the housing therebetween. In that event, the hinge arms are normally biased toward the housing to urge the flanged arms toward the aperture. As the frame member slides away from the access port, the hinge arms are pushed apart by the housing to separate the flanged arms and allow them to pass down over the side of the housing completely exposing the exterior wall and seal surface for disinfecting.

By virtue of the foregoing, there is thus provided a sample site with a flush-mounted needleless access port and a split luer arrangement so as to properly connect a male luer connector to the access port, but without obstructing wiping access to the seal member and surrounding regions of the housing for purposes of disinfection.

These and other objects and advantages of the present invention shall be made apparent from the accompanying drawings and description thereof.

### Brief Description of the Drawings

The accompanying drawings, which are incorporated in and constitute a part of this specification, illustrate embodiments of the invention and, together with a general description of the invention given above, and the detailed description given below, serve to explain the principles of the invention.
Fig. 1 is a perspective view of a first embodiment of a sample site in accordance with the principles of the present invention;
Fig. 2 is a top plan view of the sample site of Fig. 1;
Fig. 3 is a cross-sectional view of the sample site of Fig. 1 along line 3-3 of Fig. 2;
Fig. 4 is a cross-sectional view like that of Fig. 2, but with a syringe coupled thereto via a male luer connector;
Fig. 5 is a perspective view of a second embodiment of a sample site in accordance with the principles of the present invention;
Fig. 6 is a perspective view of a third embodiment of a sample site in accordance with the principles of the present invention; and
Fig. 7 is a diagrammatic view of an exemplary, closed blood sampling system incorporating a sample site of the present invention.

### Detailed Description of the Drawings

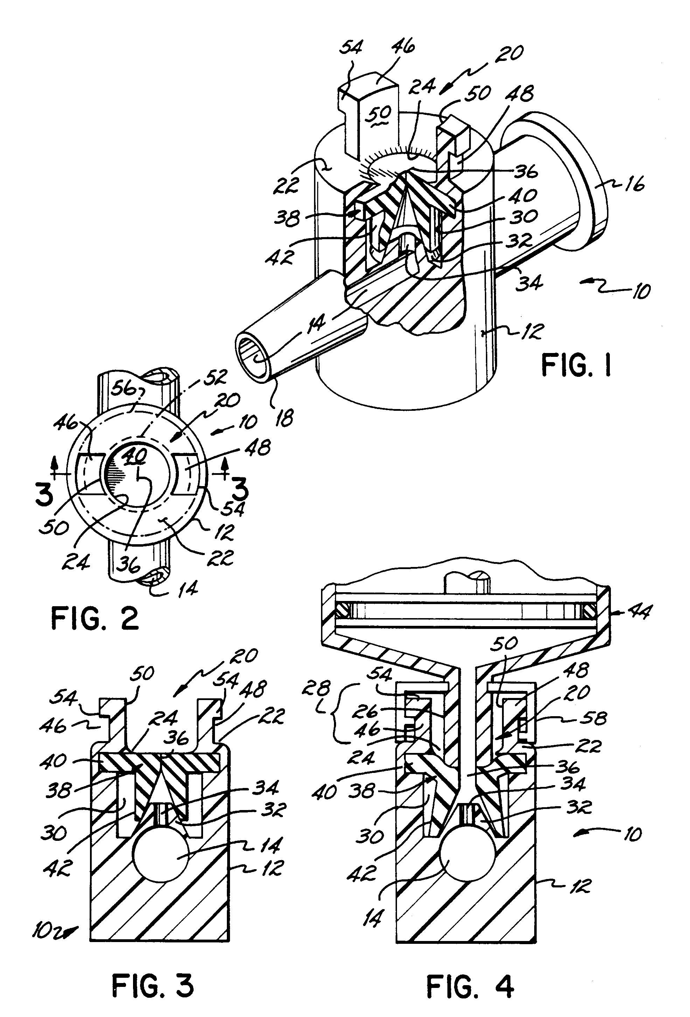

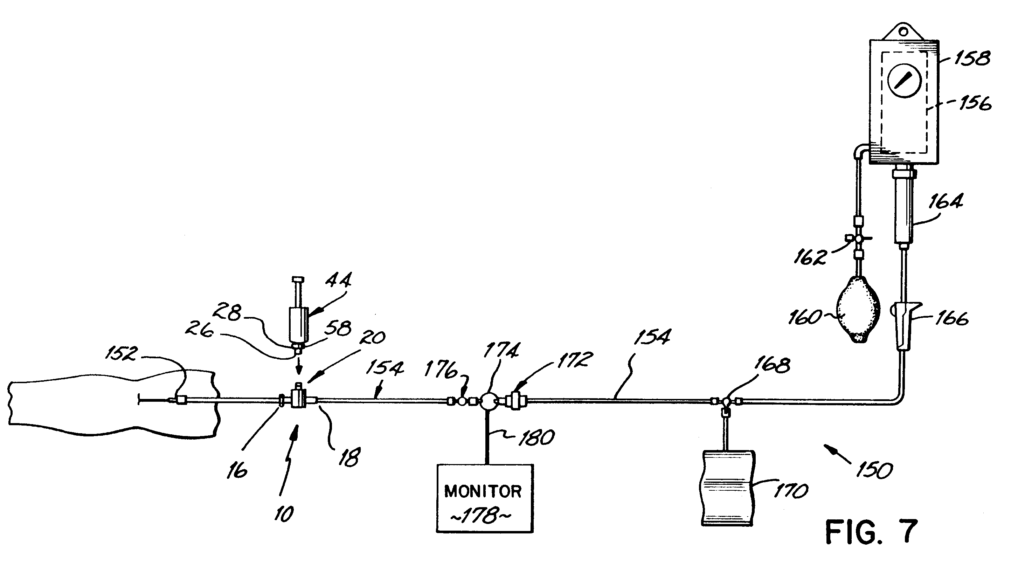

With reference to Figs. 1-4, there is shown one embodiment 10 of a sample site in accordance with the principles of the present invention. Sample site 10 is defined by a cylindrical housing 12 having a liquid path 14 extending therein. At opposite ends of path 14 are female luer connector port 16 and male taper port 18 for connection with tubing or the like, such as for fluid communication with a patient's circulatory system (see Fig. 7). Housing 12 includes a needleless connector access port 20 between ports 16 and 18 defined along generally planar, top exterior wall 22 of housing 12. Wall 22 includes an aperture 24 through which is to be received a blunt cannula such as the tip or taper 26 of a male luer connector 28 for fluid communication with liquid path 14. Aperture 24 is sized to closely surround tip 26 when received therethrough.

A valve assembly is provided at aperture 24 and within recess 30 of housing 12 between top wall 22 and liquid path 14 by frustro-conical actuating member 32 fixedly positioned within housing 12 and a seal member 38. Actuating member 32 includes a lumen 34 therethrough transverse to and in fluid communication with liquid path 14. Lumen 34 is aimed at and sealed-off by normally closed channel 36 of resilient seal member 38 positioned over actuator member 32. Seal member 38 includes hub 42 extending below seal upper surface 40 and seated over actuating member 32. The top surface 40 of seal member 38 is generally flush top wall 22 when it is closed against or adjacent aperture 24. In this regard, the thickness of wall 22 adjacent aperture 24 is relatively thin in comparison to the length of the male luer connector tip 26 so as not to define a reservoir wall relative tip 26. Exterior wall 22 may be about 0.035-0.040 inches (0.9-1.0 mm) thick such that a wipe (not shown) passed across wall 22 will easily disinfect wall 22 and surface 40 without creating a well or reservoir for contaminants or bacteria to accumulate out of reach of the wipe as it passes across top wall 22. Alternatively, surface 40 may protrude from aperture 24. Thus, it will be appreciated that upper surface 40 of seal member 38 may be considered generally flush top wall 22 in the closed position inasmuch as the surface of the valve assembly is readily accessible to be wiped with disinfectant by wiping across top wall 22 from one side to the other.

As generally described in aforesaid U.S.-A-4,874,377, under pressure from male lumen connector tip 26 against upper surface 40, seal member 38 deflects toward liquid path 14 and drives hub 42 against the conical surface of projection 32. As hub 42 rides over projection 32, channel 36 is caused to open revealing lumen 34 for fluid communication via tip 26 between liquid path 14 and an external fluidic system coupled to connector 28 such as a syringe 44 or tubing (not shown). When male luer connector 28 is removed, the resiliency of seal member 36 causes hub 42 to ride up frustro-conical actuating member 32 and reseal aperture 36 with outer surface 40 again adjacent aperture 24 and generally flush top wall 22.

To secure male luer connector 28 to sample site 10 while not otherwise obstructing wiping access to disinfect the exposed outer surface 40 of seal member 38 in aperture 24 and adjacent portions of external wall 22, a split luer arrangement is provided. To this end, extending upwardly and away from top wall 22 are a pair of flanged arms 46, 48. Arms 46, 48 are attached to top wall 22 at spaced apart locations adjacent aperture 24 such that the arcuate inner surfaces 50 of the arms define an imaginary cylinder 52 (shown in phantom line in Fig. 2) corresponding to the inner surface of a female luer connector cylinder. Similarly, flanges 54 of arms 46, 48 define an imaginary flange thread plane 56 (also shown in phantom line in Fig. 2) corresponding to the threading flange of a female luer connector onto which may be threadably received rotatable threaded cuff 58 of a male luer lock connector 28, as shown in Fig. 4. Arms 46, 48 thus define a pair of spaced apart female luer connector portions.

In the embodiment of the invention shown in Figs. 1-4, inner surface 50 and flange 54 of each arm 46, 48 define approximately fifteen percent (15%) of the circumference of a female luer connector cylinder and/or flange (a combined total of 30%) to thereby functionally provide the female luer connector functions of guiding tip 26 and locking connector 28 in place, respectively, while leaving access port 20 otherwise generally unobstructed. Thus, a clinician (not shown) may readily disinfect access port 20 and flange arms 46, 48 by wiping across the surface of top wall 22 such as in the direction from port 16 to port 18 with a disinfectant material such as with an alcohol wipe to disinfect the area about aperture 24. As a consequence, the needleless sample site of the present invention eliminates the need to use needles with their attendant risks, while greatly reducing the risk of contamination and bacterial growth normally of great concern with prior needleless sample sites.

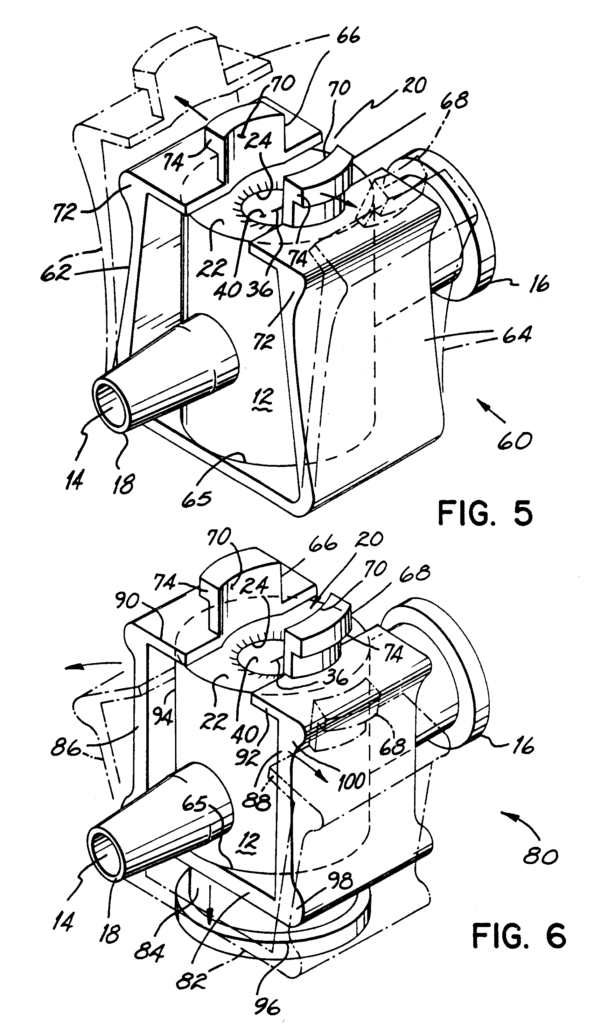

An alternative embodiment 60 of a sample site in accordance with the principles of the present invention is shown in Fig. 5. Sample site 60 is substantially similar to sample site 10 with the exception of the provision of a modified split luer connector. In the sample site of embodiment 60, the entire surface of top wall 22 may be completely freed from obstruction by placing the split luer in an inoperative position. To this end, the split luer of embodiment 60 is provided by a pair of hinge arms 62, 64 connected such as by sonic welding or glue to bottom wall 65 of housing 12 and defining at their extremities a pair of female luer connector portions or flanged arms 66, 68. Hinge arms 62, 64 may be living hinges and are normally in an inoperative position shown in dashed line in Fig. 5 with arms 66, 68 outwardly of housing 12 so as to expose the entirety of top wall 22 and seal member outer surface 40 (as well as the inner walls 70 of arms 66, 68) to be easily and readily wiped clean for purposes of disinfection.

Hinge arms 62, 64 are thickened as at 72 adjacent flanged arms 66, 68 to provide a convenient place for a clinician (not shown) to grip hinge arms 62, 64 and compress them together to bring female luer connector portions 66, 68 into an operative position shown in solid line in Fig. 5 to provide the female luer connector function for receiving a male luer connector 28 as in the case of connector portions 46, 48 described in connection with sample site 10. When male luer connector 28 is removed, hinge arms 62, 64 urge the split luer apart into the inoperative position. The inner wall 70 and flange 74 of each flanged arm 66, 68 comprise about twenty-five percent (25%) of the circumference of a female luer connector cylinder and flange, or about fifty percent (50%) total.

A yet further alternative embodiment 80 of a sample site in accordance with the present invention is shown in Fig. 6. Sample site 80 is substantially similar to sample site 70 with the exception of the mechanism for moving female luer connector portions 66, 68 between the operative and inoperative positions shown in solid and dashed line, respectively. To this end, a frame member 82 is slidably supported on an annular extension 84 attached to bottom 65 of housing 12. Attached to frame member 82 are a pair of hinge arms 86, 88 with shelves 90, 92 carrying flanged arms 66, 68, respectively. Hinge arms 86, 88 are normally biased toward side wall 94 of housing 12, such that with frame member 82 slid against bottom 65, shelves 90, 92 pass over housing top wall 22 to position flanged arms 66, 68 into the operative position adjacent aperture 24 to provide the female luer connector function. To disinfect the access port, frame member 82 is slid away from bottom 65 toward flange wall 96 of annular extension 84. As the frame member is thus moved, top wall 22 and shelves 90, 92 cooperate to urge hinge arms 86, 88 outwardly of housing 12 and away from aperture 24 allowing flanged arms 66, 68 to ride astride housing side wall 94 into the inoperative position completely exposing access port 20 for disinfecting such as by wiping a disinfectant material thereacross. Hinge arms 86, 88 are thickened top and bottom as at 98, 100 to facilitate sliding frame member 82 by finger pressure there-against in the direction desired to slide frame member 82. As shown in Fig. 6, hinge arms 86, 88 thus move in a direction parallel lumen 34 and transverse liquid path 14. The hinge arms could alternatively be mounted to slide parallel liquid path 14 and transverse lumen 34.

By virtue of the foregoing, there is provided a split liner arrangement to simulate a female luer connector, but without the reservoir which would otherwise impede ready access to access port 20 including surface 40 of seal member 38 and the adjacent annular portion of top wall 22 completely surrounding exposed surface 40 for disinfecting thereof, to reduce the likelihood that contamination or bacterial growth might occur. Although housing 12 has been shown as a T-connection, the access port and split luer combination of the present invention may be provided in other types of sample sites. Thus, the liquid path within housing 12 may be coaxial with lumen 34 rather than transverse thereto and may have only one of ports 16, 18 connected thereto. Similarly, ports 16 and 18 could form part of a Y-connector rather than a T-connector. Still further, the flush mounted needleless access port and split luer lock arrangement could also be provided at each of ports 16, 18.

Use of the sample site 10, 60 or 80 will be described in connection with a closed blood sampling system 150 as shown in Fig. 7. System 150 includes a catheter 152 for insertion into a patient's blood vessel and connected in a series via tubing 154 to a bag 156 of saline solution. Bag 156 is wrapped in a pressure cuff 158 which may be inflated by an inflation squeeze bulb 160 through a stopcock 162. The solution flows out of bag 156 through conventional drip chamber 164. A roller clamp 166 may be mounted on tubing 154 adjacent drip chamber 164 in order to selectively block the flow of solution from bag 156 toward the patient. Connected immediately downstream of roller clamp 166 is a shunted stopcock 168 coupled to a waste collection bag 170. Downstream of stopcock 168 is a flush device 172, pressure transducer 174, and three-way stopcock 176. Pressure transducer 174 is electrically connected to a monitor 178 by cable 180 for monitoring the patient's blood pressure. A transducer of the type disclosed in U.S.-A-4,920,972, the disclosure of which is fully incorporated herein by reference, may be employed. Operation of the above is described in greater detail in the aforementioned US-A-5148 811.

In use, the sample site is situated between catheter 152 and three-way stopcock 176 by coupling port 16 to catheter 152 and port 18 to stopcock 176 to continue the in-line fluid path between bag 156 and catheter 152. Syringe 44 may be connected in fluid communication therewith by first wiping access port 20 with a disinfectant and then, if they are movable, placing the female luer connector portions of the sample site into the operative position. The male luer connector 28 is then fitted to access port 20 by inserting tip 26 thereof between the inner walls of the split luer and into the aperture to deflect seal member 38 and open the valve assembly under pressure of tip 26 to reveal lumen 34 and provide a fluid path between syringe 44 and liquid path 14 coupled to catheter 152. Where the male luer connector is of the locking type, the cuff 58 thereof may be rotated onto the flanges of the split luer to maintain the connection.

After injecting or sampling as desired, syringe 44 is removed by withdrawing male luer connector tip 26, allowing seal member 38 to reclose. The access port 20 will also be accessible (or made accessible by movement of the female luer connector portions to the inoperative position) to allow access port 20 to again be disinfected by wiping thereacross.

By virtue of the foregoing, there is thus provided a needleless connector sample site in which the access port is generally unobstructed to provide wiping access for disinfecting, while eliminating the risk of needle sticks common to needle-based sample sites and to improve sterility and reduce risk of infection otherwise feared from prior needleless connector sample sites.

It will be appreciated that the sample site may be utilized as an injection site when coupled to the venous side of a patient's circulatory system, as a sample port when connected to the arterial side, or as a link to connect an exterior fluidic system to an otherwise closed fluidic system without opening the closed system, thereby avoiding the possibility of reflux in the closed system. Reference to sample site will thus be appreciated as a reference to any of the above. The blunt cannula could be shrouded or unshrouded. In the former, the shroud (not shown) may be receivable over side wall 94 of housing 12. In the latter, tip 26 may be a typical male luer slip cannula at the end of syringe 44, but without threaded cuff 58. In either event, the split luer may either be merely a pair of unflanged arms or could be eliminated altogether.

## Claims

1. A sample site comprising a housing (12), a liquid path (14) extending into the housing (12), an apertured exterior wall (22) on the housing (12) through which a blunt cannula enters the housing (12) to make fluid connection to the liquid path (14), and, seal means (38) adjacent the exterior wall aperture (24) and presenting an outer surface (40) generally flush the housing exterior wall (22) for normally sealing the aperture (24) against fluid communication with the liquid path (14), the seal means (38) being openable under pressure of the blunt cannula passing into the aperture (24) whereby to permit fluid connection thereof with the liquid path (14), the seal means (38) resealing after removal of the blunt cannula, characterised in that a pair of arms (46, 48, 66, 68) extend away from the housing (12) about the aperture (24) for receiving the blunt cannula (26) therethrough and in that the arms (46, 48, 66, 68) are spaced apart to facilitate cleaning of the seal means (38) by providing wiping access between the arms (46, 48, 66, 68) and across the seal means outer surface (40) and adjacent portions of the housing exterior wall (22).

2. A sample site as claimed in Claim 1, further comprising an actuator member (32) fixedly positioned within the housing (12) and having a lumen (34) therethrough in fluid communication with the liquid path (14), the seal means (38) being moveable into the actuator member (32) under pressure of the blunt cannula passing into the aperture (24) so as to open a channel in the seal means (38) and expose the lumen (34) therethrough to the cannula, whereby the blunt cannula on entering the housing (12) makes a fluid connection to the lumen (34) and the seal member channel being resealed with the blunt cannula removed, wherein the seal means outer surface (40) is in adjacent contact with the housing exterior wall (22).

3. A sample site as claimed in either Claim 1 or Claim 2, further comprising means (54, 74) for holding the blunt cannula (26) to the sample site (10, 60, 80).

4. A sample site as claimed in any preceding Claim, wherein the blunt cannula is a tip (26) of a male luer connector and flange means (54, 74) are provided on the arms (46, 48, 66, 68) for securing the male luer connector (28) to the sample site (10, 60, 80) with the tip (26) thereof through the aperture (24).

5. A sample site as claimed in any preceding Claim, wherein the blunt cannula is a tip (26) of a male luer connector (28) and the arms are in the form of female luer connector portions (46, 48, 50, 54, 66, 68).

6. A sample site as claimed in any preceding Claim, wherein the arms (46, 48) are fixedly connected at respective locations to the housing exterior surface (22) adjacent the aperture (24).

7. A sample site as claimed in any one of Claims 1 to 5, further comprising mounting arm means (62, 64, 86, 88, 90, 92) connected to the housing (12) remote from the aperture (24) for supporting the arms (66, 68) which in a first, inoperative, position urge the arms (66, 68) away from the aperture (24) to provide unobstructed access to the seal means outer surface (40) and an annular portion of the exterior wall (22) completely surrounding the aperture (24) and in a second position place the arms (66, 68) in operative relationship with the aperture (24) for receiving a blunt cannula (26).

8. A sample site as claimed in Claim 7, wherein the mounting arm means (62, 64) is hingedly connected to the housing (12) and normally is in the first position, the mounting arm means (62, 64) being moveable to the second position by pressure applied against the mounting arm means (62, 64).

9. A sample site as claimed in Claim 7, wherein the mounting arm means (62, 64) is slidably connected to the housing (12) and slidable relative the aperture (24), the mounting arm means (86, 88) being in the first position slid away from the aperture (24) and in the second position slid toward the aperture (24).

10. A sample site as claimed in Claim 9, wherein the housing includes a support portion (84) attached thereto and a frame member (82) slidably received over the support portion (84), the mounting arm means (86, 88) being connected to the frame member (82) for sliding movement therewith, the frame member (82) being slidable between a first position whereat the mounting arm means are in the second, operative, position and a second position whereat the mounting arms means are in the first inoperative position.

11. A sample site as claimed in Claim 10, wherein the apertured exterior wall (22) and support portion (84) are disposed respectively on opposite ends of the housing (12), the mounting arm means (86, 88) extending alongside an intermediate wall (94) therebetween, the first position of the frame member (82) being defined with the frame member (82) adjacent the intermediate wall (94) whereat the mounting arm means (86, 88) are spaced closely adjacent the intermediate wall (94), the second position of the frame member (82) being defined with the frame member (82) spaced away from the intermediate wall (94) whereat the mounting arm means (86, 88) are urged outwardly from the intermediate wall (94) and the arms (66, 68) are spaced closely adjacent the intermediate wall (94).

12. A sample site as claimed in any preceding Claim, wherein the housing includes at least one port (16, 18) coupled to the liquid path (14).

13. A sample site as claimed in any preceding Claim, wherein the housing includes first and second spaced apart ports (16, 18) at respective ends of the liquid path.

14. A sample site as claimed in Claim 13, wherein the first and second ports (16, 18) are disposed on opposite sides of the housing (12) with the apertured exterior wall (22) being positioned therebetween so as to define a T-connection.

## Patentansprüche

1. Probenahmeanordnung, umfassend ein Gehäuse (12), einen sich in das Gehäuse (12) erstreckenden Flüssigkeitsweg (14), eine gelochte Außenwand (22) an dem Gehäuse (12), durch die eine abgestumpfte Kanüle in das Gehäuse (12) eintritt, um eine Fluidverbindung zu dem Flüssigkeitsweg (14) herzustellen, und ein Verschlußmittel (38), das an die Außenwandöffnung (24) angrenzt und eine Außenfläche (40) darbietet, die im wesentlichen mit der Gehäuseaußenwand (22) bündig ist, um normalerweise die Öffnung (24) gegen die Fluidverbindung mit dem Flüssigkeitsweg (14) abzuschließen, wobei das Verschlußmittel (38) unter dem Druck der in die Öffnung (24) führenden abgestumpften Kanüle zu öffnen ist, um dadurch die Fluidverbindung derselben mit dem Flüssigkeitsweg (14) zu ermöglichen, das Verschlußmittel (38) nach dem Entfernen der abgestumpften Kanüle wieder schließt, gekennzeichnet dadurch, daß ein Paar Arme (46, 48, 66, 68) zum Aufnehmen der abgestumpften Kanüle (26) sich darin um die Öffnung (24) weg von dem Gehäuse (12) erstreckt, und daß die Arme (46, 48, 66, 68) voneinander beabstandet sind, um das Reinigen des Verschlußmittels (38) durch Vorsehen eines Wischzuganges zwischen den Armen (46, 48, 66, 68) und über der Verschlußmittelaußenfläche (40) und angrenzenden Teilen der Gehäuseaußenwand (22) zu erleichtern.

2. Probenahmeanordnung nach Anspruch 1, die außerdem ein Betätigungselement (32) umfaßt, das fest in dem Gehäuse (12) angeordnet ist und ein Lumen (34) dorthindurch besitzt, das in Fluidverbindung mit dem Flüssigkeitsweg (14) steht, wobei das Verschlußelement (38) unter dem Druck der in die Öffnung (24) führenden abgestumpften Kanüle in das Betätigungselement (32) bewegbar ist, um einen Kanal in dem Verschlußelement (38) zu öffnen und das Lumen (34) dadurch zu der Kanüle freizugeben, wobei die abgestumpfte Kanüle beim Eintreten in das Gehäuse (12) eine Fluidverbindung zu dem Lumen (34) herstellt und der Verschlußelementkanal mit entfernter abgestumpfter Kanüle wieder geschlossen wird, wobei die Verschlußelementaußenfläche (40) in anliegendem Kontakt mit der Gehäuseaußenwand (22) ist.

3. Probenahmeanordnung nach Anspruch 1 oder 2, die außerdem Mittel (54, 74) zum Halten der abgestumpften Kanüle (26) an der Probenahmeanordnung (10, 60, 80) umfaßt.

4. Probenahmeanordnung nach einem vorhergehenden Anspruch, bei der die abgestumpfte Kanüle eine Spitze (26) eines Luer-Einsteckanschlußstückes ist und Flanschmittel (54, 74) an den Armen (46, 48, 66, 68) zum Sichern des Luer-Einsteckanschlußstückes (28) an der Probenahmeanordnung (10, 60, 80) mit seiner durch die Öffnung (24) geführten Spitze (26) vorgesehen sind.

5. Probenahmeanordnung nach einem vorhergehenden Anspruch, bei der die abgestumpfte Kanüle eine Spitze (26) eines Luer-Einsteckanschlußstückes (28) ist und die Arme die Form von Luer-Aufnahmeanschlußstückteilen (46, 48, 50, 54, 66, 68) haben.

6. Probenahmeanordnung nach einem vorhergehenden Anspruch, bei der die Arme (46, 48) fest an entsprechenden Stellen der Gehäuseaußenfläche (22) an der Öffnung (24) angrenzend verbunden sind.

7. Probenahmeanordnung nach einem der Ansprüche 1 bis 5, die außerdem Befestigungsarmmittel (62, 64, 86, 88, 90, 92) umfaßt, die zum Tragen der Arme (66, 68) an dem Gehäuse (12) entfernt von der Öffnung (24) angeschlossen sind, die in einer ersten, inaktiven Position die Arme (66, 68) weg von der Öffnung (24) drücken, um einen hindernisfreien Zugang zu der Verschlußmittelaußenfläche (40) und einem die Öffnung (24) vollständig umgebenden, ringförmigen Teil der Außenwand (22) vorzusehen, und in einer zweiten Position die Arme (66, 68) in aktive Beziehung zu der Öffnung (24) zum Aufnehmen einer abgestumpften Kanüle (26) bringen.

8. Probenahmeanordnung nach Anspruch 7, bei der das Befestigungsarmmittel (62, 64) an dem Gehäuse (12) schwenkbar befestigt und normalerweise in der ersten Position ist, wobei das Befestigungsarmmittel (62, 64) durch gegen das Befestigungsarmmittel (62, 64) aufgebrachten Druck in die zweite Position bewegbar ist.

9. Probenahmeanordnung nach Anspruch 7, bei der das Befestigungsarmmittel (62, 64) an dem Gehäuse (12) verschiebbar angeschlossen und in bezug auf die Öffnung (24) verschiebbar ist, wobei das Befestigungsarmmittel (86, 88) in der ersten Position von der Öffnung (24) geschoben und in der zweiten Position zu der Öffnung (24) geschoben ist.

10. Probenahmeanordnung nach Anspruch 9, bei der das Gehäuse ein daran befestigtes Trägerteil (84) und ein Rahmenelement (82) umfaßt, das über dem Trägerteil (84) verschiebbar aufgenommen ist, wobei Befestigungsarmmittel (86, 88) an dem Rahmenelement (82) zur Gleitbewegung mit diesem verbunden sind, das Rahmenelement (82) zwischen einer ersten Position, in der die Befestigungsarmmittel in der zweiten, aktiven Position sind, und einer zweiten Position, in der die Befestigungsarmmittel in der ersten inaktiven Position sind, verschiebbar ist.

11. Probenahmeanordnung nach Anspruch 10, bei der die gelochte Außenwand (22) und das Trägerteil (84) jeweils auf gegenüberliegenden Enden des Gehäuses (12) angeordnet sind, wobei sich die Befestigungsarmmittel (86, 88) entlang einer dazwischenliegenden Zwischenwand (94) erstrecken, die erste Position des Rahmenelementes (82) mit dem Rahmenelement (82) an die Zwischenwand (94) angrenzend definiert wird, in der die Befestigungsarmmittel (86, 88) dicht neben der Zwischenwand (94) beabstandet sind, die zweite Position des Rahmenelementes (82) mit dem Rahmenelement (82) von der Zwischenwand (94) beabstandet definiert wird, in der die Befestigungsarmmittel (86, 88) von der Zwischenwand (94) nach außen gedrückt werden und die Arme (66, 68) dicht neben der Zwischenwand (94) beabstandet sind.

12. Probenahmeanordnung nach einem vorhergehenden Anspruch, bei der das Gehäuse mindestens eine mit dem Flüssigkeitsweg (14) verbundene Öffnung (16, 18) umfaßt.

13. Probenahmeanordnung nach einem vorhergehenden Anspruch, bei der das Gehäuse erste und zweite, voneinander beabstandete Öffnungen (16, 18) an entsprechenden Enden des Flüssigkeitsweges umfaßt.

14. Probenahmeanordnung nach Anspruch 13, bei der die ersten und zweiten Öffnungen (16, 18) auf gegenüberliegenden Seiten des Gehäuses (12) angeordnet sind, wobei die gelochte Außenwand (22) dazwischen angeordnet ist, um eine T-Verbindung zu definieren.

## Revendications

1. Poste d'échantillonnage comprenant un boîtier (12), un chemin de liquide (14) s'étendant dans le boîtier (12), une paroi extérieure (22) présentant, sur le boîtier (12), une ouverture à travers laquelle une canule épointée entre dans le boîtier (12) pour établir une connexion hydraulique avec le chemin de liquide (14), et un moyen d'étanchéité (38) adjacent à l'ouverture de paroi extérieure (24) et présentant une surface extérieure (40) globalement alignée avec la paroi extérieure de boîtier (22), pour isoler normalement l'ouverture (24) contre toute communication hydraulique avec le chemin de liquide (14), le moyen d'étanchéité (38) pouvant être ouvert sous pression de la canule épointée passant dans l'ouverture (24), de manière à permettre une connexion hydraulique de ce dernier avec le chemin de liquide (14), le moyen d'étanchéité (38) rétablissant une étanchéité après l'enlèvement de la canule épointée, caractérisé en ce qu'un couple de bras (46, 48, 66, 68) s'étend en s'éloignant du boîtier (12) autour de l'ouverture (24), pour loger en leur sein la canule épointée (26), et en ce que les bras (46, 48, 66, 68) sont écartés entre eux, pour faciliter le nettoyage des moyens d'étanchéité (38), en fournissant un accès de balayage entre les bras (46, 48, 66, 68) et sur la surface extérieure du moyen d'étanchéité (40), et des parties adjacentes de la paroi extérieure de boîtier (22).

2. Poste d'échantillonnage selon la revendication 1, comprenant en outre un organe d'actionnement (32) placé rigidement dans le boîtier (12) et ayant dans son sein un lumen (34) en communication hydraulique avec le chemin de liquide (14), le moyen d'étanchéité (38) étant déplaçable dans l'organe d'actionnement (32), sous la pression de la canule épointée passant dans l'ouverture (24), de manière à ouvrir un canal dans le moyen d'étanchéité (38) et a exposer le lumen (34) s'y trouvant à la canule, de manière que la canule épointée, lorsqu'elle entre dans la boîtier (12), établisse une connexion hydraulique avec le lumen (34) et le canal d'organe d'étanchéité de nouveau isolé lorsque la canule épointée est retirée, dans lequel la surface extérieure de moyen d'étanchéité (40) est en contact intime avec la paroi extérieure de boîtier (22).

3. Poste d'échantillonnage selon la revendication 1 ou 2, comprenant en outre des moyens (54, 74) pour maintenir la canule épointée (26) sur le poste d'échantillonnage (10, 60, 80).

4. Poste d'échantillonnage selon l'une des revendications précédentes, dans lequel la canule épointée est un embout (26) d'un connecteur à cône de Luer mâle (28) et des moyens formant bride (54, 74) sont prévus sur les bras (46, 48, 66, 68) pour fixer le connecteur à cône de Luer mâle (28) au poste d'échantillonnage (10, 60, 80), par son embout (26) dans l'ouverture (24).

5. poste d'échantillonnage selon l'une des revendications précédentes, dans lequel la canule épointée est un embout (26) d'un connecteur à cône de Luer mâle (28) et les bras se présentent sous la forme de partie de connexion à cône de Luer femelle (46, 48, 66, 68).

6. Poste d'échantillonnage selon l'une des revendications précédentes, dans lequel les bras (46, 48) sont connectés rigidement en des emplacements respectifs à la surface extérieure de boîtier (22), de manière adjacente à l'ouverture (24).

7. Poste d'échantillonnage selon l'une des revendications 1 à 5, comprenant en outre des moyens formant bras de montage (62, 64, 86, 88, 90, 91) connectés au boîtier (12), à distance de l'ouverture (24) pour supporter les bras (66, 68) qui, dans une première position non fonctionnelle, poussent les bras (66, 68) à s'éloigner de l'ouverture (24) pour fournir un accès non obstrué à la surface extérieure de moyen d'étanchéité (40) et à une partie annulaire de la paroi extérieure (22) entourant complètement l'ouverture (24) et, dans une deuxième position, pousse les bras (66, 68) en relation fonctionnelle avec l'ouverture (24), pour recevoir une canule épointée (26).

8. Poste d'échantillonnage selon la revendication 7, dans lequel le moyen formant bras de montage (62, 64) est connecté de manière articulé au boîtier (12) et se trouve normalement dans la première position, le moyen formant bras de montage (62, 64) pouvant être déplacé vers la deuxième position, sous l'effet d'une pression appliquée sur le moyen formant bras de montage (62, 64).

9. Poste d'échantillonnage selon la revendication 7, dans lequel le moyen formant bras de montage (62, 64) est connecté de manière coulissante au boîtier (12) et est coulissant par rapport l'ouverture (24), le moyen formant bras de montage (86, 88) se trouvant, dans la première position, coulissée à l'écart de l'ouverture (24) et, dans la position, coulissemée vers l'ouverture (24).

10. Poste d'échantillonnage selon la revendication 9, dans lequel le boîtier comprend une partie de support (24) lui étant fixée et un organe formant cadre (82) logé coulissant sur la partie de support (84), le moyen formant bras de montage (86, 88) étant connecté à l'organe formant cadre (82) pour effectuer un mouvement coulissant conjointement avec ce dernier, l'organe formant cadre (82) étant coulissant entre une première position faisant que les moyens formant bras de montage se trouvent dans la deuxième position, fonctionnelle, et une deuxième position faisant que les moyens formant bras de montage se trouvent dans la première position, non fonctionnelle.

11. Poste d'échantillonnage selon la revendication 10, dans lequel la paroi extérieure (22) à ouverture et la partie de support (84) sont respectivement disposées sur les extrémités opposées du boîtier (12), le moyen formant bras de montage (86, 88) s'étendant latéralement le long d'une paroi intermédiaire (94), située entre ces derniers, la première position de l'organe formant cadre (82) étant définie, tandis que l'organe formant cadre (82) est adjacent à la paroi intermédiaire (94), faisant que les moyens formant bras de montage (86, 88) sont espacés de manière intimement adjacente à la paroi intermédiaire (94), la deuxième position de l'organe formant cadre (82) étant définie, tandis que l'organe formant cadre (82) est espacé de la paroi intermédiaire (94), faisant que les moyens formant bras de montage (86, 88) sont poussés vers l'extérieur de la paroi intermédiaire (94), et les bras (66, 68) sont espacés de manière intimement adjacentes à la paroi intermédiaire (94).

12. poste d'échantillonnage selon l'une quelconque des revendications précédentes, dans lequel le boîtier comprend au moins un orifice (16, 18) couplé au chemin de liquide (14).

13. poste d'échantillonnage selon l'une quelconque des revendications précédentes, dans lequel le boîtier comprend des premier et deuxième orifices (16, 18) espacés entre eux, aux extrémités respectives du chemin de liquide.

14. poste d'échantillonnage selon la revendication 13, dans lequel les premier et deuxième orifices (16, 18) sont disposés sur les côtés opposés du boîtier (12), tandis que la paroi extérieure (22) à ouverture est placée entre eux de manière à définir une connexion en T.
